# EUROPEAN PATENT SPECIFICATION

(11) **EP 3 289 098 B1**
(45) Date of publication and mention of the grant of the patent: **20.03.2024**
(21) Application number: 16720619.2
(22) Date of filing: 15.04.2016
(51) Int. Cl.: C12Q 1/68

(54) **METHODS TO REDUCE EVAPORATION DURING ELEVATED TEMPERATURE**
VERFAHREN ZUR VERDAMPFUNGSVERRINGERUNG BEI ERHÖHTER TEMPERATUR
PROCÉDÉS DE RÉDUCTION DE L'ÉVAPORATION EN CAS DE TEMPÉRATURES ÉLEVÉES

(30) Priority: 30.04.2015 US 201614701043
(43) Date of publication of application: 07.03.2018
(73) Proprietor: Sakura Finetek U.S.A., Inc., Torrance, CA 90501 (US)
(72) Inventor: YAMANISHI, Douglas T., Redondo Beach, California 90278 (US); HOM, Clifford, Redondo Beach, California 90277 (US); SHAH, Amit D., Rancho Palos Verdes, California 90275 (US)
(74) Representative: Studio Torta S.p.A.
(86) International application number: PCT/US2016/027945
(87) International publication number: WO 2017/180160

(56) References cited:
- WO-A1-01/16172
- WO-A1-94/02644
- WO-A1-94/02646
- WO-A1-95/05147
- WO-A1-2012/055981
- WO-A1-2014/144663
- CN-A- 103 013 639
- CN-A- 104 531 397
- JP-A- 2014 018 127
- US-A- 6 127 188
- US-A1- 2004 235 104
- US-A1- 2007 048 770
- US-A1- 2010 080 955
- US-A1- 2014 224 655
- US-A1- 2014 242 595
- R. P. VAN GIJLSWIJK ET AL: "Improved localization of fluorescent tyramides for fluorescence in situ hybridization using dextran sulfate and polyvinyl alcohol.", JOURNAL OF HISTOCHEMISTRY AND CYTOCHEMISTRY, vol. 44, no. 4, 1 April 1996 (1996-04-01), pages 389-392, XP055278459, US ISSN: 0022-1554, DOI: 10.1177/44.4.8601698
- HAMIL K G ET AL: "Follicle-stimulating hormone regulation of AP-1: inhibition of c-jun and stimulation of jun-B gene transcription in the rat Sertoli cell", MOLECULAR AND CELLULAR ENDOCRINOLOGY, ELSEVIER IRELAND LTD, IE, vol. 99, no. 2, 1 March 1994 (1994-03-01), pages 269-277, XP025826740, ISSN: 0303-7207, DOI: 10.1016/0303-7207(94)90017-5 [retrieved on 1994-03-01]
- AUVINEN E ET AL: "Polyethylene glycol increases specificity of hybridization typing of HPV specimens", MOLECULAR AND CELLULAR PROBES, ACADEMIC PRESS, LONDON, GB, vol. 3, no. 3, 1 September 1989 (1989-09-01), pages 289-298, XP024865870, ISSN: 0890-8508, DOI: 10.1016/0890-8508(89)90009-1 [retrieved on 1989-09-01]

## Description

### FIELD

Method to reduce evaporation in processing a tissue or cellular sample.

### BACKGROUND

Immunohistochemical assays and assay techniques based on in situ hybridization are widely used in medical diagnostics such as to diagnose abnormal cells such as those found in cancerous tumors or to diagnose another disease. Many assays involve the addition of heat to the sample, such as to a sample in a reagent on a slide. Assay steps at elevated temperatures can cause substantial evaporation of assay reagents. When small volumes are used, sample denaturation, hybridization, wash and aging steps and the resulting assay can be compromised by evaporation.

US 2007/048770 describes methods and processing reagents for improving washing and aging of a biological sample in an assay. The processing reagents comprise an aqueous base reagent and a low vapor pressure composition such as glycerol, propylene glycol and ethylene glycol in sufficient amount to raise the boiling point of the base reagent. Amounts of 10 percent to 40 percent by volume are described as useful for washing and aging related to fluorescent in situ hybridization (FISH) and in situ hybridization (ISH) assays.

### DETAILED DESCRIPTION

A reagent and a method of use of a reagent including an evaporation reducing agent in an aqueous solution operable to reduce evaporation of the reagent in processing of a tissue or cellular sample is disclosed. Representative processing for which a reagent as described finds use include, but are not limited to, dewaxing, cell conditioning/antigen retrieval/cell aging, peroxide/phosphatase block, probe denature, probe hybridization, washing, linker hybridization, antibody incubation, probe detection, chromogen precipitation and counterstain involving an elevated temperature step (e.g. an elevated temperature step in an in situ hybridization procedure). The solution can representatively be used in denaturation, wash or hybridization steps of a nucleic acid (single or double stranded probe) hybridization assay or in an incubation, antigen retrieval or a wash step using an antibody in immunohistochemical or immunocytochemical staining. A suitable reagent includes other components such as a detergent/surfactant which helps with solution spreading in a hybridization solution reagent. A suitable amount of an evaporation reducing agent in a reagent operable for tissue or cellular processing is an amount that will limit a loss of the reagent due to evaporation to 20 percent or less under industry acceptable reagent processing conditions such as subjecting a hybridization solution to an elevated temperature between 25°C and 50°C for 10 minutes to 24 hours (e.g., a hybridization or incubation process) or to an elevated temperature of between 60°C and 100°C for two to ninety minutes (e.g., a denaturing or antigen retrieval process). In one embodiment, a suitable amount of an evaporation reducing agent is in the range of 11 percent to 60 percent by volume of a regent composition (e.g., solution).

In one embodiment, a reagent (composition) includes an effective evaporation reducing amount of an evaporation reducing agent selected from ethylene glycol, glycine, serine, isoethionic acid, ethanolamineand 1,3-propanediol. In one embodiment, a suitable reagent includes one or more evaporation reducing agents (e.g., a reagent includes a combination of two or three evaporation reducing agents.

An evaporation reducing agent may be in a molecular free form (a molecule) or an acceptable salt thereof (a compound) or ionic liquid. An example of a salt is a hydrohalide salt of the molecule such as a hydrochloride salt.

In one embodiment, an evaporation reducing agent as a solute reduces the vapor pressure of a solution upon its mixing with one or more other reagents. Such solute could be a molecule, an ionic salt, an ionic liquid, a non-ionic agent that may exhibit complete or partial solubility or miscibility with water and is capable of reducing a partial pressure of an aqueous solution to which it is mixed or otherwise introduced. Additionally, an evaporation reducing agent may or may not form an azeotropic mixture with water. If an evaporation reducing agent does form an azeotropic mixture with water, the solute, in one embodiment, forms a negative azeotrope with water.

Generally speaking, when a solvent and a solute are mixed in this context, a resulting chemical potential of the solvent is lowered so that a partial pressure of the solvent molecules is reduced and, relative to a solvent-only solution, has a reduced tendency to transition into gas phase. Representatively, a partial pressure of water in an aqueous solution where a solute is a nonvolatile evaporation reducing agent will be reduced which would result in elevation of a boiling point of the mixture as well as a reduction in evaporation at the surface.

Hydrogen bonding can occur when in a molecule, a hydrogen atom is attached to an electronegative atom such as an oxygen, nitrogen or fluorine atom and the molecule comes in proximity with an electropositive atom. The electrostatic attraction of this nature has a strength on the order of 5-30kJ/mol which is stronger than Van der Waals attraction but weaker than covalent or ionic bonding.

In one embodiment, an evaporation reducing agent is mixed as a solute with water to reduce a vapor pressure of the water by creating a solution where a partial pressure of the water is reduced such that an assay (e.g., an immunohistologic assay) can be carried out more efficiently than if the partial pressure was not lowered. In one embodiment, a solute is selected such that there is no or minimum residue left behind which may interfere with an assay known to a person skilled in the art,

In one embodiment, a reagent operable for use in processing a cellular or tissue sample is prepared by combining a base reagent and an evaporation reducing agent. Additional components may also be combined in a suitable composition. The reagent can be added to an aqueous buffer solution to reduce evaporation during elevated temperatures. In one embodiment, the evaporation reducing agent is combined in an amount to limit any loss of the combination due to evaporation to 20 percent or less and, in another embodiment, to 10 percent or less. Representatively, an evaporation reducing agent or combination of reducing agents is/are present in a composition in an amount between 11 percent and 60 percent by volume or an amount between 1 percent and 60 percent of the reducing agent is a non-glycol-containing compound. Following a combination of a base reagent, evaporation reducing agent and any other components, the composition may be mixed.
In one embodiment, a method operable in processing of a paraffin-free or deparaffinized tissue or cellular sample for use in an assay at an elevated temperature is disclosed. In one embodiment, a method is operable to reduce evaporation of any aqueous based processing reagent. The method includes contacting a tissue or cellular sample with an aqueous composition comprising a hybridization solution, a detergent/surfactant and at least one evaporation reducing agent selected from an ethylene glycol, a glycine, a serine, an isoethionic acid, an ethanolamine and a 1,3-propanediolSuch contact includes applying the reagent to a sample on a slide. Following contacting the sample with the reagent, the sample may be processed according to techniques known in the art.

In one embodiment, a method operable in processing of a paraffin-free or deparaffinized tissue or cellular sample for use in an assay at an elevated temperature is disclosed. In one embodiment, a method is operable to reduce evaporation of any aqueous based processing reagent by adding a solute to a solvent. The resulting chemical potential of the solvent is lowered so that the partial pressure of the solvent molecules is reduced and they have lesser tendency to turn into gas phase. Partial pressure of water in an aqueous solution where solute is a nonvolatile substance is reduced which would result in elevation of boiling point as well as reduction in evaporation at the surface.

### EXAMPLES

### Example 1: Volume loss due to evaporation during oligonucleotide hybridization at different hybridization temperatures.

400 µl of hybridization solution (reagent) was applied to each of six sample slides and the slides were individually heated to different temperatures for 30 minutes. It was observed that evaporation occurred on the slide with hybridization solution during the hybridization step.

**Table 1. Effect of temperature on evaporation**

| Hybridization Temperature (°C) | Ave volume loss due to evaporation (µl) |
|---|---|
| 25 | 7.5 |
| 35 | 37.5 |
| 37 | 50.0 |
| 39 | 47.5 |
| 41 | 50.0 |
| 43 | 55.0 |

### Example 2: Reducing evaporation during oligonucleotide hybridization by different reagents.

400 µl of hybridization solution (reagent) with different evaporation reducing agents was applied to respective sample slides and heated to 43° C for 30 minutes. One sample slide included a hybridization solution with no evaporation reducing agent. It was observed that a significantly greater evaporation of the hybridization solution occurred with the sample slide containing a hybridization solution without an evaporation reducing agent during the 43° C hybridization step but was reduced with the addition of an evaporation reducing agent.

**Table 2. Ability of added agent to hybridization solution (reagent) to reduce evaporation**

| Reagent | Avg volume loss (µl) |
|---|---|
| No addition | 55.0 |
| 20% Ethylene Glycol | 20.0 |
| 20% Propylene Glycol | 45.0 |
| 20% Glycerol | 42.5 |
| 20% Polyethylene Glycol | 15.0 |

### Example 3: Reducing evaporation using different concentrations of ethylene glycol during hybridization.

400 µl of hybridization solution (reagent) having different concentrations of an evaporation reducing agent of ethylene glycol was applied to respective sample slides and heated to 43° C for 30 minutes. For comparison, 400 µl of hybridization solution without ethylene glycol was also applied to a sample slide and heated to 43° C for 30 minutes. It was observed that evaporation was reduced with the addition of ethylene glycol to the hybridization solution. The assay performance for the hybridization solution containing ethylene glycol was found to be similar to hybridization solution without ethylene glycol.

**Table 3. Reduction in volume loss in % ethylene glycol (EG) in hybridization solution**

| | | Comparison to Starting Volume | |
|---|---|---|---|
| % EG | Starting vol (µl) | Avg evaporatiion (µl) | % Evaporation |
| 0% | 400 | 48 | 12% |
| 7% | 400 | 28 | 7% |
| 14% | 400 | 18 | 4% |
| 20% | 400 | 13 | 3% |
| 27% | 400 | 10 | 2% |
| 33% | 400 | 0 | 0% |
| 40% | 400 | 0 | 0% |

### Example 4: Reproducibility in reducing evaporation volume loss during hybridization.

400 µl of hybridization solution with specific concentrations of an evaporation reducing agent of ethylene glycol was applied to each sample slide and heated to 43° C for 30 minutes. For comparison, 400 µl of hybridization solution was applied to each sample slide and heated to 43°C for 30 minutes. It was observed that evaporation was reduced with the addition of ethylene glycol to the hybridization solution.

**Table 4. Reduction in evaporation due to addition of ethylene glycol**

| | oligonucleotide hybridization solution with 0% ethylene glycol | | oligonucleotide hybridization solution with 20% ethylene glycol | |
|---|---|---|---|---|
| Slide | Starting vol (µl) | Evaporation (µl) | Starting vol (µl) | Evaporation (µl) |
| 1 | 400 | 45 | 400 | 12 |
| 2 | 400 | 50 | 400 | 15 |
| 3 | 400 | 45 | 400 | 15 |
| 4 | 400 | 55 | 400 | 17 |
| 5 | 400 | 40 | 400 | 15 |
| 6 | 400 | 50 | 400 | 10 |
| 7 | 400 | 50 | 400 | 12 |
| 8 | 400 | 45 | 400 | 15 |
| 9 | 400 | 50 | 400 | 12 |
| 10 | 400 | 45 | 400 | 12 |
| | **Avg** | **48** | **Avg** | **14** |

### Example 5: Reduction in evaporation during denaturation.

About 400 µl of hybridization solution with an evaporation reducing agent of 20 percent ethylene glycol was applied to each sample slide and heated to 92 - 98° C for 2 minutes. For comparison, about 400 µl of hybridization solution without ethylene glycol was applied to each sample slide and heated to 92 - 98° C for 2 minutes. It was observed that evaporation was reduced with the addition of ethylene glycol to the hybridization solution.

**Table 5. Reduction in evaporation with addition of ethylene glycol to hybridization solution during denaturation.**

| | | oligonucleotide hybridization solution with 0% ethylene glycol | | | oligonucleotide hybridization solution with 20% ethylene glycol | | |
|---|---|---|---|---|---|---|---|
| Temp (°C) | Time (min) | Starting vol (µl) | Evaporation (µl) | Avg. loss due to Evaporation | Starting vol (µl) | Evaporation (µl) | Avg. loss due to Evaporation |
| 92 | 2 | 400 | 60 | 15% | 400 | 15 | 4% |
| 92 | 2 | 400 | 65 | | 400 | 17 | |
| 92 | 2 | 400 | 50 | | 400 | 15 | |
| 95 | 2 | 400 | 60 | 16% | 400 | 30 | 7% |
| 95 | 2 | 400 | 70 | | 400 | 30 | |
| 95 | 2 | 400 | 65 | | 400 | 25 | |
| 98 | 2 | 400 | 80 | 19% | 400 | 33 | 8% |
| 98 | 2 | 400 | 75 | | 400 | 30 | |
| 98 | 2 | 400 | 75 | | 400 | 30 | |

### Example 6: Reduction in evaporation during antibody incubation step.

About 400 µl of primary antibody diluent (reagent) with an evaporation reducing agent of 20 percent ethylene glycol was applied to each sample slide and incubated at a specific temperature for 30 minutes. For comparison, about 400 µl of primary antibody diluent without ethylene glycol was applied to each sample slide and incubated at a specific temperature for 30 minutes. It was observed that evaporation was reduced with the addition of ethylene glycol to the antibody diluent.

**Table 6. Reduction in evaporation with addition of ethylene glycol to antibody diluent**

| | Primary antibody diluent with 0% ethylene glycol | | | Primary antibody diluent with 20% ethylene glycol | | |
|---|---|---|---|---|---|---|
| Temp (°C) | Starting vol (µl) | Evaporation (µl) | Avg. loss due to Evaporation | Starting vol (µl) | Evaporation (µl) | Avg. loss due to Evaporation |
| 20 | 400 | 0 | 0% | 400 | 0 | 0% |
| 20 | 400 | 0 | | 400 | 0 | |
| 25 | 400 | 3 | 1% | 400 | 0 | 0% |
| 25 | 400 | 5 | | 400 | 0 | |
| 31 | 400 | 30 | 9% | 400 | 5 | 1% |
| 31 | 400 | 40 | | 400 | 5 | |
| 37 | 400 | 60 | 17% | 400 | 3 | 1% |
| 37 | 400 | 75 | | 400 | 5 | |
| 43 | 400 | 95 | 23% | 400 | 20 | 6% |
| 43 | 400 | 90 | | 400 | 25 | |

### Example 7: Reducing evaporation during oligonucleotide hybridization by different reagents.

400 µl of hybridization solution with different evaporation reducing agents was applied to respective sample slides and heated to 43°C for 30 minutes. A hybridization solution containing no evaporation reducing agent was also applied to a sample slide. It was observed that evaporation occurred in the slide with hybridization solution during the 43°C hybridization step but was reduced with the addition of specific reagent.

**Table 7. Ability of added evaporation reducing agent to hybridization solution to reduce evaporation**

| Evaporation Reducing Agent | Avg volume loss (µl) | |
|---|---|---|
| No addition | 57.5 | |
| 20% ethylene glycol | 20 | |
| 20% glycine | 27.5 | |
| 20% serine | 17.5 | |
| 20% isoethionic acid | 25 | |
| 20% ethanolamine | 17.5 | |
| 20% 1,3-propanediol | 22.5 | |

### Example 8: Reducing evaporation using ethylene glycol during hybridization at different temperatures.

400 µl of hybridization solution (reagent) with ethylene glycol was applied to respective sample slides and heated to 35, 37 or 40° C for 120 minutes. For comparison, 400 µl of hybridization solution without ethylene glycol was also applied to a sample slide and heated to 40° C for 120 minutes. It was observed that evaporation was reduced with the addition of ethylene glycol to the hybridization solution. The assay performance for the hybridization solution containing ethylene glycol at different hybridization temperatures was found to be similar to hybridization solution without ethylene glycol.

### Reagents

### Hybridization solution

| Chemical | Final conc. |
|---|---|
| SSC | 2 x |
| Tris-HCl | 10 mM |
| Dextran sulfate | 10 % |
| Denhardt's solution | 2 x |
| Tween | 0.05 % |
| Triton | 0.10 % |
| Salmon sperm DNA | 50 µg/ml |
| Evaporation reducing agent | varied |
| water | varied |

### Primary antibody diluent

| Chemical | Final conc. |
|---|---|
| Sodium Azide | 0.05% |
| Sodium Chloride | 300 mM |
| Sodium Phosphate Dibasic | 8 mM |
| Potassium Phosphate monobasic | 2 mM |
| Green Color Dye | 0.025% |
| BSA Powder | 1% |
| Tris Base | 10 mM |
| Tris HCl | 40 mM |
| Tween | 0.05% |
| Triton | 0.1% |
| Evaporation reducing agent | varied |
| water | varied |

In the description above, for the purposes of explanation, numerous specific details have been set forth in order to provide a thorough understanding of the embodiments. It will be apparent however, to one skilled in the art, that one or more other embodiments may be practiced without some of these specific details. The particular embodiments described are not provided to limit the invention but to illustrate it. The scope of the invention is not to be determined by the specific examples provided above but only by the claims below. In other instances, well-known structures, devices, and operations have been shown in block diagram form or without detail in order to avoid obscuring the understanding of the description. Where considered appropriate, reference numerals or terminal portions of reference numerals have been repeated among the figures to indicate corresponding or analogous elements, which may optionally have similar characteristics.

## Claims

1. A method of processing a tissue or cellular sample using a reagent including an evaporation reducing agent in an aqueous solution operable to reduce evaporation of the reagent in processing the tissue or cellular sample comprising:
contacting a tissue or cellular sample on a slide with an aqueous composition comprising a hybridization solution, a detergent/surfactant and at least one evaporation reducing agent selected from ethylene glycol, glycine, serine, isoethionic acid, ethanolamine and 1,3-propanediol and wherein the at least one evaporation reducing agent is present in an amount of one percent by volume or greater when the at least one evaporation reducing agent is a non-glycol-containing agent or an amount of 11 percent by volume or greater when the at least one evaporation reducing agent is a glycol-containing agent, the percent by volume being with respect to the total volume of the composition; and
processing the tissue or cellular sample at a temperature between 25°C and 50°C for 10 minutes to 24 hours or between 60°C and 100°C for two to ninety minutes.

2. The method of claim 1, wherein the evaporation reducing agent is present in an amount up to 60 percent by volume.

3. The method of claim 1, wherein the at least one evaporation reducing agent comprises ethylene glycol.

4. The method of claim 1, wherein the at least one evaporation reducing agent comprises one of glycine, serine, isoethionic acid, ethanolamine or 1,3-propanediol.

5. The method of claim 1, wherein the aqueous composition comprises more than one of the evaporation reducing agents.

6. Use of a reagent for the processing of a tissue or cellular sample on a slide at a temperature between 25°C and 50°C for 10 minutes to 24 hours or between 60°C and 100°C for two to ninety minutes, the reagent comprising an aqueous composition of a hybridization solution, a detergent/surfactant and an evaporation reducing agent selected from ethylene glycol, glycine, serine, isoethionic acid, ethanolamine and 1,3-propanediol present in an amount of one percent by volume or greater when the evaporation reducing agent is a non-glycol-containing agent or an amount of 11 percent by volume or greater when the evaporation reducing agent is a glycol-containing agent, the percent by volume being with respect to the total volume of the composition.

7. The reagent of claim 6, wherein the evaporation reducing agent is present in an amount up to 60 percent by volume.

8. The reagent of claim 6, wherein the aqueous composition comprises more than one of the evaporation reducing agents.

## Patentansprüche

1. Ein Verfahren zur Bearbeitung einer Gewebe- oder Zellprobe unter Verwendung eines Reagens, das ein die Verdampfung verringerndes Mittel in einer wässrigen Lösung enthält, das dazu geeignet ist, die Verdampfung des Reagens bei der Bearbeitung der Gewebe- oder Zellprobe zu verringern, wobei das Verfahren aufweist:
Bringen einer Gewebe- oder Zellprobe auf einem Objektträger in Kontakt mit eine wässrigen Mischung, die eine Hybridisierungslösung, ein Detergens/Tensid und mindestens ein die Verdampfung verringerndes Mittel aufweist, ausgewählt aus Ethylenglykol, Glycin, Serin, Isethionsäure, Ethanolamin und 1,3-Propanediol, und wobei das mindestens eine verdampfungsverringernde Mittel in einer Menge von einem Volumenprozent oder mehr vorhanden ist, wenn das mindestens eine verdampfungsverringernde Mittel ein nicht glykolhaltiges Mittel ist, oder in einer Menge von 11 Volumenprozent oder mehr vorhanden ist, wenn das mindestens eine verdampfungsverringernde Mittel ein glykolhaltiges Mittel ist, wobei sich die Volumenprozente auf das Gesamtvolumen der Mischung beziehen; und
Bearbeiten der Gewebe- oder Zellprobe bei einer Temperatur zwischen 25 °C und 50 °C für 10 Minuten bis 24 Stunden oder zwischen 60 °C und 100 °C für zwei bis neunzig Minuten.

2. Das Verfahren nach Anspruch 1, wobei das verdampfungsverringernde Mittel in einer Menge von bis zu 60 Volumenprozent vorhanden ist.

3. Das Verfahren nach Anspruch 1, wobei das mindestens eine verdampfungsverringernde Mittel Ethylenglykol aufweist.

4. Das Verfahren nach Anspruch 1, wobei das mindestens eine verdampfungsverringernde Mittel eines von Glycin, Serin, Isethionsäure, Ethanolamin, oder 1,3-Propanediol aufweist.

5. Das Verfahren nach Anspruch 1, wobei die wässrige Mischung mehr als eines der verdampfungsverringernden Mittel aufweist.

6. Verwendung eines Reagens zur Bearbeitung einer Gewebe- oder Zellprobe auf einem Objektträger bei einer Temperatur zwischen 25 °C und 50 °C für 10 Minuten bis 24 Stunden oder zwischen 60 °C und 100 °C für zwei bis neunzig Minuten, wobei das Reagens aufweist: eine wässrige Mischung einer Hybridisierungslösung, eines Detergens/Tensids und mindestens eines verdampfungsverringernden Mittels, ausgewählt aus Ethylenglykol, Glycin, Serin, Isethionsäure, Ethanolamin und 1,3-Propanediol, wobei das mindestens eine verdampfungsverringernde Mittel in einer Menge von einem Volumenprozent oder mehr vorhanden ist, wenn das mindestens eine verdampfungsverringernde Mittel ein nicht glykolhaltiges Mittel ist, oder in einer Menge von 11 Volumenprozent oder mehr vorhanden ist, wenn das mindestens eine verdampfungsverringernde Mittel ein glykolhaltiges Mittel ist, wobei sich die Volumenprozente auf das Gesamtvolumen der Mischung beziehen.

7. Das Reagens von Anspruch 6, wobei das verdampfungsverringernde Mittel in einer Menge von 60 Volumenprozent vorhanden ist.

8. Das Reagens von Anspruch 6, wobei die wässrige Mischung mehr als eines der verdampfungsverringernden Mittel aufweist.

## Revendications

1. Méthode de traitement d'un échantillon tissulaire ou cellulaire utilisant un réactif comportant un agent de réduction d'évaporation dans une solution aqueuse utilisable pour réduire l'évaporation du réactif dans le traitement de l'échantillon tissulaire ou cellulaire comprenant :
la mise en contact d'un échantillon tissulaire ou cellulaire sur une lame avec une composition aqueuse comprenant une solution d'hybridation, un détergent/tensio-actif et au moins un agent de réduction d'évaporation sélectionné parmi l'éthylène glycol, la glycine, la sérine, l'acide iséthionique, l'éthanolamine et 1,3-propanediol et dans laquelle l'au moins un agent de réduction d'évaporation est présent dans une quantité d'un pour cent en volume ou plus lorsque l'au moins un agent de réduction d'évaporation est un agent ne contenant pas de glycol ou une quantité de 11 pour cent en volume ou plus lorsque l'au moins un agent de réduction d'évaporation est un agent contenant du glycol, le pourcentage en volume étant par rapport au volume total de la composition ; et
le traitement de l'échantillon tissulaire ou cellulaire à une température entre 25 °C et 50 °C pendant 10 minutes à 24 heures ou entre 60 °C et 100 °C pendant deux à quatre-vingt-dix minutes.

2. Méthode selon la revendication 1, dans laquelle l'agent de réduction d'évaporation est présent dans une quantité jusqu'à 60 pour cent en volume.

3. Méthode selon la revendication 1, dans laquelle l'au moins un agent de réduction d'évaporation comprend de l'éthylène glycol.

4. Méthode selon la revendication 1, dans laquelle l'agent de réduction d'évaporation comprend un parmi la glycine, la sérine, l'acide iséthionique, l'éthanolamine et 1,3-propanediol l.

5. Méthode selon la revendication 1, dans laquelle la composition aqueuse comprend plus d'un des agents de réduction d'évaporation.

6. Utilisation d'un réactif pour le traitement d'un échantillon tissulaire ou cellulaire sur une lame à une température entre 25 °C et 50 °C pendant 10 minutes à 24 heures ou entre 60 °C et 100 °C pendant deux à quatre-vingt-dix minutes, le réactif comprenant une composition aqueuse d'une solution d'hybridation, un détergent/tensio-actif et un agent de réduction d'évaporation sélectionné parmi l'éthylène glycol, la glycine, la glycine, la sérine, l'acide iséthionique, l'éthanolamine et 1,3-propanediol présent dans une quantité d'un pour cent en volume ou plus lorsque l'agent de réduction d'évaporation est un agent ne contenant pas de glycol ou une quantité de 11 pour cent en volume ou plus lorsque l'agent de réduction d'évaporation est un agent contenant du glycol, le pourcentage en volume étant par rapport au volume total de la composition.

7. Réactif selon la revendication 6, dans lequel l'agent de réduction d'évaporation est présent dans une quantité jusqu'à 60 pour cent en volume.

8. Réactif selon la revendication 6, dans lequel la composition aqueuse comprend plus d'un des agents de réduction d'évaporation.
